Europäisches Patentamt

European Patent Office

Office européen des brevets

(11) Publication number: **0 338 453**
**A2**

# EUROPEAN PATENT APPLICATION

(21) Application number: **89106730.8**

(22) Date of filing: **14.04.89**

(51) Int. Cl.⁴: **C12N 7/00 , A61K 37/02**

(30) Priority: **18.04.88 IT 2022788**

(43) Date of publication of application:
**25.10.89 Bulletin 89/43**

(84) Designated Contracting States:
**AT BE CH DE ES FR GB GR IT LI LU NL SE**

(71) Applicant: **CO PHARMA CORPORATION S.R.L.**
**Via Assarotti, 52**
**I-16123 Genova(IT)**

(72) Inventor: **Filice, Gaetano**
**Via S. Ulderico, 2**
**Pavia(IT)**
Inventor: **Cereda, Paolo Martino**
**Via Loatti, 17**
**Pavia(IT)**
Inventor: **Cornaglia Ferraris, Paolo**
**Piazza Manin, 2**
**Genova(IT)**

(74) Representative: **Minoja, Fabrizio**
**Studio Consulenza Brevettuale Via Rossini,**
**8**
**I-20122 Milano(IT)**

(54) **A method of sensitization of small animals to the infection of human retroviruses.**

(57) A method of sensitization of small laboratory animals such as rabbits, guinea pigs, rats and mice to retroviruses consists of inducing in the animals aseptic peritonitis and subsequently inoculating the free virus or cells infected by the virus in the presence of interleukin-2 (IL-2).

EP 0 338 453 A2

# A METHOD OF SENSITIZATION OF SMALL ANIMALS TO THE INFECTION OF HUMAN RETROVIRUSES

The present invention relates to a method of sensitization of small laboratory or wild animals to the infection by retroviruses, particularly human immunodeficiency virus (HIV) and the animals sensitized to said viruses to be used for in vivo drug or vaccine testing.

The health problems connected with the syndromes of acquired immunodeficiency (AIDS) are well-known: the prospect of expansion of this epidemic in the world population in the next few years is worrisome and has stimulated substantial efforts in research directed to the development of vaccines. One of the main requirements for the preparation and study of vaccines is the availability of a suitable animal model which permits to evaluate the possible toxicity and the efficacy of the preparations. Up to the present time the chimpanzee is the only animal species which may be infected by the HIV virus which is the etiologic agent of AIDS (Gajdusek DC, et al. Infection of Chimpanzee By Human T-lymphotropic Retroviruses In Brain And Other Tissues From AIDS Patients.·Lancet 1:55-56, 1985; Fults PN, et al. Persistent Infection Of Chimpanzees With Human T-lymphotropic Virus Type III/lymphadenopathy Associated Virus: A Potential Model For Acquired Immunodeficiency Syndrome. J. Virology 58:116, 1986).

Unfortunately, the chimpanzee is not an ideal model because it does not develop the disease called AIDS but presents only some immunological interactions. In spite of this fact, the chimpanzee is a suitable model to analyze the degree of protection imparted by vaccination after exposure to the free virus and/or to the infected cells. The major problems connected with the study of a vaccine for AIDS with the chimpanzee model are: (1) the limited number of animals; (2) the high cost; (3) handling difficulties; and (4) the designation of the protected species.

Several species of small animals, such as mice, rats, hamsters, guinea pigs, rabbits and shrew-mice have been inoculated with HIV or infected cells, but in every case no infection has been reported. (Morrow, WJW, et al. Small Animals Are Not Susceptible To Human Immunodeficiency Virus Infection. J gen virol 68:2253-2257, 1987).

Therefore, the most common laboratory animals, such as mice, rats, rabbits, guinea pigs, hamsters, cats, dogs and the like, are not considered at present suitable for the development of a model for the pre-clinical study of vaccines to HIV.

It is therefore an object of the present invention to provide an efficient method of infecting animals, other than primates, with the human immunodeficiency virus (HIV).

It is also an object of this invention to provide a low-cost animal model for the study of AIDS and related diseases.

It is further an object of the present invention to provide a low cost, efficient method for determining the effectiveness of the therapeutic agents, such as vaccines in preventing or minimizing retroviral infections, such as human immunodeficiency virus (HIV) infections, including AIDS or AIDS Related Complex (ARC).

These and other objects of the present invention will be apparent to those of ordinary skill in the art in light of the present specification.

The method according to the present invention consists of inducing in small animals an aseptic peritonitis, and subsequently administering to the treated animals an effective amount of a retrovirus in combination with an effective amount of interleukin-2 (IL-2) and optionally of phytohemoagglutinin (PHA).

A further aspect of this invention concerns a test kit comprising an effective amount of an agent capable of inducing aseptic peritonitis, and effective amounts of retrovirus and Interleukin-2.

This invention further provides a method of determining the effectiveness of a vaccine in preventing infection caused by a retrovirus in an animal comprising administering to the animal an effective amount of the vaccine; then treating the animal with an amount of an agent effective to induce aseptic peritonitis; administering to the animal so treated effective amounts of retrovirus and Interleukin-2; and assaying said animal for the presence or absence of retrovirus infection, thereby determining the effectiveness of the vaccine.

The infections produced according to the method of the present invention include various diseases caused by exposure to retroviruses, e.g., human retroviruses, including human immunodeficiency virus (HIV), responsible for the disease known such as AIDS and ARC. In addition, various disease infections caused by exposure to simian immunodeficiency virus (SIV), and may also be provoked in animals according to the method of this invention, by substituting the causative simian agent for the retrovirus or HIV. Such simian immunodeficiency viruses are also known to cause disease in humans.

As used herein the term "human immunodeficiency virus (HIV)" is intended to comprise various serotypes of the retrovirus including types HIV-I, HIV-II, and HIV-III. These retroviral species are also referred to as Human T-cell Lymphotrophic Virus (HTLV) e.g., HTLV-I, HTLV-II and HTLV-III.

The method of infecting an animal with human immunodeficiency virus may also be carried out by administering the human immunodeficiency virus and Interleukin-2 (IL-2) in combination with a mitogen or mitogenic compound, e.g., a T-cell mitogen, Such mitogenic compounds include, for example, phytohemagglutinin (PHA), Concanavalin A (Con A), pokeweed mitogen (PWM) and lipopolysaccharides (LPS), and these may also be used in combination with each other. Preferred as a mitogenic compound is PHA in a pharmaceutically acceptable carrier, such as RPMI 1640.

Useful in the practice of the present invention is the mitogenic agent, phytohemagglutinin or PHA, which is a protein extracted from the red kidney bean. PHA is commercially available from the Sigma Chemical Corp., P.O. Box 14508, St. Louis, Missouri, U.S.A.

Concanavalin A (Con A) is a plant protein which is isolated from the jack bean and is also commercially available from Miles-Yeda Ltd.

PWM and LPS are also commercially available from various sources. For example, pokeweed mitogen (PWM) may be obtained from United States Biochemical Corporation, P.O. Box 22400, Cleveland, Ohio 44122.

The animals which may be sensitized according to the present invention are: rabbits, rats, mice, hamsters, guinea pigs, shrew-mice, cats, dogs. Particularly preferred for the ease of usage and handling are rabbits, rats and mice. The method according to the invention permits to induce infection by HIV or other retroviruses with 100% efficiency. The serum conversion in the animals being studied has resulted persistent in the period of time up to more than one year after the infection. The infection which has occurred after HIV exposure has been determined by means of serologic analyses for the purpose of determining the presence of anti-HIV antibodies and p24. The specificity of the antibodies has been confirmed by means of the competitive ELISA test utilizing polyvalent anti-HIV human serum, anti-p24, gp41 and gp120 monospecific purified sheep antibodies. The specificity of the p24 antigen has been confirmed by means of ELISA neutralization test utilizing polyvalent anti-HIV human serum. Two to six months after the infection, the removal of the peripheral blood lymphocytes (PBL) from the animals under study was carried out. The presence of the p24 antigen was evaluated in the supernatant of the culture of macrophages by means of the ELISA test. The infected cells have been identified by utilizing anti-HIV antibodies conjugated with peroxidase or monoclonal anti-p17, p24, gp120 antibodies of HIV in immunofluorescence tests. The viruses isolated have been propagated in suitable cells (H9, CEM or human lymphocytes) and the lymphocytes have been co-cultivated with the same cells. Then the evaluation of the p24 antigen has been made in the supernatant by means of the ELISA test. The supernatants of the cultures have been subjected to the test of reverse transcriptase activity. The infected cells are identified by using anti-HIV antibodies conjugated with peroxidase and monoclonal anti-p17, p24 and gp 120 antibodies in the immunofluorescence tests.

The presence of retroviruses in the cultures has been verified by electron-microscopy while the isolated viruses have been propagated in H9 cells (Science 224, 497, 1984). The isolated viruses have been inoculated intraperitoneally according to the same procedure in a second group of animals which then have been subjected to serologic analysis of the infection and virus re-isolation from PBL as described hereinabove. The molecular analysis of the genomes of the HIV-inoculated virus and the virus isolated from the animals has been carried out by hybridization of DNA extracted from the infected cells and treated with endonuclease and with HIV probes labelled with radioactive phosphorus. The proteins of the inoculated HIV virus and the isolated virus have been analyzed by electrophoretic separation in polyacrylamide gel (SDS-PAGE) of the sediments obtained after centrifugation of the supernatants of the infected cells and coloring the proteins with Comassie blue. The proteins have been identified with Western blot utilizing strips containing the proteins of the two viruses, transferred in the electrical field and the monoclonal anti-p24, gp41, and gp120 antibodies, human polyvalent anti-HIV serum and monospecific serum of rabbit directed towards the immuno-dominating epitopes of the pericapside (env9). The re-isolated virus of the infected rodents appears to be totally identical to the human HIV.

The induction of the aseptic peritonitis is carried out according to known procedures. For instance, by the intraperitoneal administration of agents, such as thioglycolate, tuftsin, talcum, homogenates of Listeria monocytogenes, Freund adjuvant, sodium metaperiodate, sodium caseinate, shellfish, glycogen type II, klelsiella oxitoca glycoprotein (Biostim[R] Sharper, Milan, Italy), in the presence or absence of peptides which activate the phagocytosis, such as the substance P. It is particularly preferred to use physiological solutions of concentration 2-5% of thioglycolate to be administered depending upon the animals in the amount of 1-10 ml.

4% Thioglycollate (Merck GmbH, Germany) in distilled water is usually administered in doses from 0.5 to 2 ml in rabbits.

Other methods of inducing aseptic peritonitis proved to be equally effective: for instance, thioglycollate could be substituted by or added with a talc suspension in sterile PBS (usually 2 ml of a 1-10% by weight suspension of talc for mice; 40 ml for rats and hamsters and 50 ml for Guinea pigs). Heparin will be preferably added at the dose of 5 U/ml.

A pre-treatment of animals with compounds able to increase the efficacy of the phagocytic cells may also be convenient. For instance, the compounds disclosed in US-4567182 may be conveniently administered to mice at doses ranging from 1 to 50 mg/kg body weight, subcutaneously. A suitable administration protocol is 1 mg/kg x 10 days; 5 mg/kg x 10 days, 25 mg/kg x 7 days, 50 mg/kg per 3 days. The last dose is preferably injected i.p. together with the infecting virus.

The experimental infection is carried out a few days after the induction of the aseptic peritonitis, usually after 4 or 5 days, utilizing preferably cells infected by the virus HIV or the free virus present in the supernatant of the cultures of the infected cells, in the presence of recombinant interleukin-2 in a concentration 0.1-1 mcg/ml and PHA (1-10 mcg/ml).

The PHA may optionally also be injected intraperitoneally into the animals, in amounts from 10 to 100 mg in sterile isotonic saline. An amount of about 35 μg in 2 ml of sterile isotonic saline proved to be suitable for the administration to mice whereas rabbits needed higher amounts, usually 50 μg or higher. Concanavalin A (Miles-Yeda Ltd.) may be optionally used in substitution to or in combination with PHA, at doses from 1 to 200 μg. A dose of 50 μg in 2.0 ml of sterile isotonic saline was found to be suited for mice infection.

In case of direct HIV infection, suitable ranges of infecting doses are from $0.9 \times 10^{-5}$ infection units/animal to $2.2 \times 10^{-5}$ infection units/animal. When infected cells are used, from 3 to $10 \times 10^6$ cells are administered to animals, in a value from 0.5 to 2 ml per animal, according to the animal size.

The present invention also provides a test kit for infecting an animal with a retrovirus, e.g., human immunodeficiency virus (HIV), comprising an effective amount of agent capable of inducing aseptic peritonitis, and effective amounts of a retrovirus, e.g., human immunodeficiency virus, and Interleukin-2.

The various aspects discussed hereinabove with respect to the method of infecting an animal with a retrovirus virus apply as well as to this test kit. Thus, for example, a mitogenic compound, such as phytohemagglutinin, Concanavalin A, pokeweed mitogen and a lipopolysaccharide, or a combination of any of the foregoing may also be included. The retrovirus is intended to include human immunodeficiency virus (HIV), including the following serotypes: HIV-I, HIV-II and HIV-III.

In addition, the agent in such a kit which induces aseptic peritonitis, may be selected from thioglycolate, tuftsin, talcum, homogenates of Listeria monocytogenes, Freunds adjuvant, sodium metaperiodate, sodium caseinate, shellfish, glycogen type II and Klebsiella oxytocia glycoprotein.

Furthermore, the type of animal that can be infected using such a kit has been described hereinabove. The routes of administration, the effective amounts of each of the above described agents, virus, Interleukin-2, and mitogenic compounds have all been described above and may be employed in the test kit of the present invention. These may all be varied according to the particular subject animal and the retrovirus of interest.

The method according to the invention permits to inoculate known quantities of the retrovirus or cells infected by the retrovirus in animals previously treated with vaccines for an accurate evaluation of the protection. The monitoring of the animals then may be easily carried out by frequently collecting the samples and with very reduced costs. In addition it is possible to evaluate the efficacy of prophylactic treatment of antiviral pharmaceutical preparations in the animals inoculated with known quantites of the virus. The method according to the invention is further illustrated hereinbelow with specific examples using rabbits and mice, but the example is not intended to be limiting.

### EXAMPLE 1

#### Infection of the Rabbits

a) Aseptic peritonitis has been induced by inoculation of 1.8 ml of a 4% thioglycolate (Merck, Darmstadt) solution in sterile water in eleven rabbits. One rabbit died due to septic peritonits which followed after the perforation of one intestinal ansa. After four days three rabbits have been inoculated with 1x10e7 cells infected with HIV (H9/HIV obtained from the laboratory of Dr. R.C. Gallo), in the presence of 0.5 mcg/ml of recombinant interleukin-2 (IL-2) and 5 mcg/ml of PHA (Sigma Chemical Corp. USA); a second group of four animals has been injected with free virus (the supernatat of H9/HIV) cells and IL-2 (0.5 mcg/ml) and the remaining three animals have been injected with 1x10e7 cells not infected (H9) and IL-2 (0.5 mcg/ml).

#### Determination of the Infection

## (a) Serologic Analyses

After fourteen days anti-HIV antibodies and antigens of HIV have been found in the sera obtained from the animals infected in the ELISA test. The specificity of the antibodies has been demonstrated in competitive tests and the specificity of the antibodies HIV has been demonstrated with neutralization tests. After about one year the animals still are positive in antibodies and antigens, although the values have decreased.

## (b) Viral Isolation

Six months after inoculation the lymphocytes of the peripheric blood from two of the three rabbits which had been inoculated with cells infected by H9.HIV have been collected and placed in a long term culture. The adhering mononucleate cells (macrophages) released free HIV antigens seven days after cultivation. The immunoperoxidase tests have shown a minimum percentage of positive cells. The viral protection of positive cells is minimum. The viral production has increased after co-cultivation with H9 cells. The cells which did not adhere (lymphocytes) after co-cultivation with H9 cells released in the supernatant HIV antigens. The virions with the typical human retrovirus morphology have been observed by means of an electronic microscope. About 3-5% of the lymphocytes have shown to be peroxidase positive. More than 100,000 cpm/ml of reverse transcriptase activity have been found in the supernatants of the cultures.

## (c) In Vivo Transmission

One rabbit has been inoculated with the virus isolated from the rabbits (1x10e7 H9/HIV-R1). Anti-HIV antibodies and anti-HIV antigens have been found 25 days later, and the positivity has been retained for more than 150 days. After 14 days a retrovirus (HIV-R2) has been isolated from the PBL removed from the rabbit which had been inoculated with HIV-R1 virus after co-cultivation with H9 cells. Recently two rabbits have been inoculated with the virus HIV-R2 (1x10e7 cells H9/HIV-R2), and after two weeks the serum conversion has been found.

## EXAMPLE 2

Infection of mice

Mice were infected by the same procedure of example 1, using $2.10^6$ H9-HIV-III$_B$ infected cells/mouse (the O.D. of the supernatant detected by p24 antigen were $\geq$ 2; the R.T. 90.000 cpm/min). Aseptic peritonitis was previously induced in mice by administration of 1 ml of a 4% thioglycollate solution per animal. A commercial ELISA test (Behring -Enzignost-HIV-micro$^R$) was used to detect the presence of IgG antibodies to HIV. All the infected animals gave positive results to the ELISA test.

## Claims

1. A method of sensitization of small animals to the human retroviruses which consists of inducing in said animals an aseptic peritonitis and subsequently inoculating the free retrovirus or cells infected by the retro virus in the presence of interleukin-2 and optionally of phytohemoagglutinin and/or concanavalin A.

2. The method according to claim 1 wherein said animals are rabbits, rats, mice, guinea pigs, hamsters, shrew-mice, cats and dogs.

3. The invention according to claim 1, wherein said infection is induced by inoculation of cells infected by the virus or by the free virus present in the supernatant or cells infected by the virus.

4. The method according to claim 1 wherein said interleukin is present in the cultures of the infected cells or in the viral suspension in the amount of 0.1-1 mcg/ml and phytohemoagglutinin in the amount of 1-10 mcg/ml.

5. The method according to claim 4 wherein said interleukin is present in the amount of 0.5 mcg/ml.

6. The method according to claim 1, wherein said aseptic peritonitis is induced by administration of thioglycolate, tuftsin, talcum, homogenates of Listeria monocytogenes, Freund adjuvant, sodium metaperiodate, sodium caseinate, shellfish, glycogen type II, Klebsiella oxitoca glycoprotein.

7. The method according to any one of the previous claims, wherein the virus is the human immunodeficiency virus (HIV).

8. A test kit comprising an effective amount of an agent capable of inducing aseptic peritonitis, and effective amounts of a retrovirus and Interleukin-2.

9. A method of determining in an animal the effectiveness of a vaccine in preventing infection caused by a retrovirus comprising:

a) administering to the animal an effective amount of the vaccine;

b) then treating the animal with an amount of an agent effective to induce aseptic peritonitis;

c) administering to the animal so treated effective amounts of retrovirus and Interleukin-2; and

d) assaying said animal for the presence or absence of retrovirus infection, thereby determining the effectiveness of the vaccine.

10. Small animals for experimental purposes sensitized to the infection of the human immunodeficiency virus (HIV).